# FASCICULE DE BREVET EUROPEEN

(11) **EP 4 559 401 B1**
(45) Date de publication et mention de la délivrance du brevet: **27.05.2026**
(21) Numéro de dépôt: 24214701.5
(22) Date de dépôt: 22.11.2024
(51) Int. Cl.: A61B 6/00

(54) **SYSTEME D'IMAGERIE MEDICALE MODULABLE**
SKALIERBARES MEDIZINISCHES BILDGEBUNGSSYSTEM
MODULATABLE MEDICAL IMAGING SYSTEM

(30) Priorité: 23.11.2023 FR 2312966
(43) Date de publication de la demande: 28.05.2025
(73) Titulaire: THALES, 92190 Meudon (FR); BA HEALTHCARE, 35740 Pace (FR)
(72) Inventeur: NUZZO, Stefania, 78140 VELIZY-VILLACOUBLAY (FR); ROYER, Guillaume, 78140 VELIZY-VILLACOUBLAY (FR); GUIRIMAND, Jean-Baptiste, 78140 VELIZY-VILLACOUBLAY (FR); COUSTAUD, Nicolas, 78140 VELIZY-VILLACOUBLAY (FR); POTTIER-VINCENT, David, 35740 PACE (FR); RIEFFEL, François, 35740 PACE (FR)
(74) Mandataire: Atout PI Laplace

(56) Documents cités:
- CN-A- 87 100 501
- US-A- 5 388 142
- US-A1- 2011 317 817

## Description

L'invention se rapporte à un système d'imagerie médicale.

Dans le domaine médical, il peut être difficile voire impossible pour un patient de se déplacer jusqu'aux outils ou systèmes permettant de réaliser une imagerie médicale. Il existe alors des systèmes d'imagerie médicale de l'art antérieur permettant à un professionnel médical de se déplacer jusqu'au patient afin de réaliser l'imagerie médicale.

Dans ce contexte, on connaît des systèmes tels que celui décrit dans le document EP3064140. Il s'agit de systèmes d'imagerie médicale munis de roues permettant leur déplacement sur de courtes distances (typiquement de chambre en chambre au sein d'un hôpital).

Ce type de systèmes est lourd, encombrant et difficile à transporter. Pour ces raisons, ces systèmes sont utilisés uniquement en un seul lieu au sein duquel ils sont éventuellement déplacés, notamment le milieu hospitalier/médical, mais ne sont pas déplacés ou transportés à l'extérieur de ce lieu.

Il existe également d'autres systèmes d'imagerie médicale transportables moins lourds qui peuvent être transportés dans différents lieux. Ces systèmes restent néanmoins soit suffisamment lourds et encombrants pour nécessiter l'utilisation d'un grand véhicule tel qu'une camionnette pour leur transport, soit pour les plus légers, insuffisamment puissants pour ne permettre qu'un nombre d'examens limité (radiographie d'un membre uniquement...).

Le brevet CN 87100501 A décrit un système d'imagerie radiologique mobile composé d'un support démontable sur lequel sont fixés séparément le tube à rayons X et le détecteur. Les deux modules sont reliés au support par des connecteurs électromécaniques monoblocs (guides-tiges, fiches à ressort) qui assurent un assemblage précis et empêchent le fonctionnement tant que les pièces ne sont pas correctement positionnées, garantissant ainsi sécurité et protection contre les vibrations pendant le transport.

L'invention vise à fournir un système d'imagerie médicale à la fois facilement transportable et suffisamment puissant pour permettre de réaliser un large panel d'examens médicaux.

L'invention propose à cet effet un système d'imagerie médicale comportant :
- un support comportant un cadre ;
- au moins deux roues assemblées directement ou indirectement au cadre de sorte à permettre le déplacement dudit support ;
- un dispositif d'imagerie médicale ;
- un dispositif de commande configuré pour commander le dispositif d'imagerie médicale ; et
- un dispositif d'alimentation électrique configuré pour alimenter en énergie électrique le dispositif d'imagerie médicale et/ou le dispositif de commande,

le dispositif d'imagerie médicale, le dispositif de commande et le dispositif d'alimentation électrique étant configurés pour être chacun assemblé au support de manière amovible,
le support comportant en outre deux longerons assemblés au cadre et configurés pour pivoter par rapport au cadre entre une position de fonctionnement et une position de rangement, le système d'imagerie médicale comportant en outre une source de rayons X, un détecteur de rayons X et une valise, la valise étant dimensionnée pour loger la source de rayons X et/ou le détecteur de rayons X, la valise présentant une largeur sensiblement égale à une distance entre les longerons.

On entend par assemblage et notamment par assemblage amovible le fait de réunir ou lier des éléments ensemble de manière démontable. Cet assemblage peut être par exemple un assemblage mécanique (type fixation démontable, emboitement élastique, clipsage, disposition dans un logement ou sur une sur surface de réception) ou encore un assemblage magnétique. Grâce à l'assemblage de manière amovible, le système d'imagerie médicale selon l'invention est modulable. Cela permet de faciliter le transport puisque les différentes parties du système d'imagerie médicale peuvent être séparément manipulées et rangées par exemple dans un véhicule pour le transport. Le système d'imagerie médicale ainsi configuré peut être typiquement transporté dans un coffre de voiture standard (notamment de type voiture compacte ou berline).

Grâce à la modularité du système d'imagerie médicale il n'est pas nécessaire de faire de concession sur la puissance électrique, une puissante batterie pouvant être utilisée.

La modularité du système d'imagerie médicale permet également, en cas de panne par exemple, de remplacer ou réparer une seule partie ou module du système et d'intervenir facilement pour le remplacement ou la réparation.

La structure du support avec des roues permet par ailleurs de faciliter le déplacement et le transport du système d'imagerie médicale. Le support portant les autres parties du système d'imagerie médicales (le dispositif d'imagerie médicale, le dispositif de commande et le dispositif d'alimentation électrique) peut être tiré ou poussé.

Le système d'imagerie médicale est avantageusement compact et léger. De préférence, le système d'imagerie médicale pèse moins de 60 kilogrammes.

Des caractéristiques préférées particulièrement commodes du système d'imagerie médicale selon l'invention sont présentées ci-dessous.

Le système d'imagerie médicale peut comporter en outre un boîtier configuré pour être assemblé au support de manière amovible.

Les roues du système d'imagerie médicale peuvent être assemblées au cadre de manière amovible.

Les roues du système d'imagerie médicale peuvent être assemblées à une base, ladite base étant assemblée de manière amovible au cadre.

Le cadre comporte deux montants et une portion de préhension reliant les montants.

Les longerons en position de fonctionnement peuvent s'étendre sensiblement horizontalement, les longerons en position de rangement étant alors parallèles aux montants du cadre et s'étendant entre lesdits montants.

Chaque longeron peut comprendre une roue au niveau d'une extrémité libre.

Le support peut comporter en outre une plaque de réception configurée pour recevoir le dispositif d'alimentation électrique, la plaque de réception étant assemblée au cadre et configurée pour pivoter par rapport au cadre entre une position de fonctionnement et une position de rangement, la plaque de réception en position de fonctionnement s'étendant sensiblement horizontalement, la plaque de réception en position de rangement étant orthogonale aux montants du cadre et s'étendant sensiblement verticalement.

La plaque de réception peut comprendre au moins une roue sur une face inférieure.

La source de rayons X peut présenter une limite supérieure de charge de tube supérieure ou égale à 40 mAs.

Le support peut comprendre un logement configuré pour recevoir le détecteur de rayons X.

Le support peut comporter un bras assemblé au cadre et ayant une extrémité libre configurée pour recevoir la source de rayons X.

Le bras peut comprendre un tronçon avant et un tronçon arrière articulés l'un par rapport à l'autre, le bras étant mobile entre une position de service et une position escamotée, le tronçon avant et le tronçon arrière formant ensemble un angle non nul, de préférence supérieur à 30°, lorsque le bras est en position de service, le tronçon avant et le tronçon arrière étant repliés l'un sur l'autre lorsque le bras est en position escamotée.

Le système d'imagerie médicale peut comporter en outre un actionneur assemblé au bras et configuré pour déplacer le bras entre la position de service et la position escamotée, l'actionneur étant de préférence un vérin électrique.

D'autres particularités et avantages de l'invention apparaîtront encore dans la description ci-après en référence aux dessins annexés, donnés à titre d'exemples non limitatifs:
- la figure 1 représente une partie d'un système d'imagerie médicale selon un mode de réalisation de l'invention ;
- la figure 2 représente un support du système d'imagerie médicale dans une première configuration ;
- la figure 3 représente le support dans une deuxième configuration ou configuration de rangement ;
- la figure 4 représente le système d'imagerie médicale dans une troisième configuration ou configuration de déplacement ;
- les figures 5 et 6 représentent le système d'imagerie médicale en cours de déploiement à partir de la configuration de rangement ; et
- la figure 7 représente le système d'imagerie médicale dans une quatrième configuration ou configuration de déploiement.

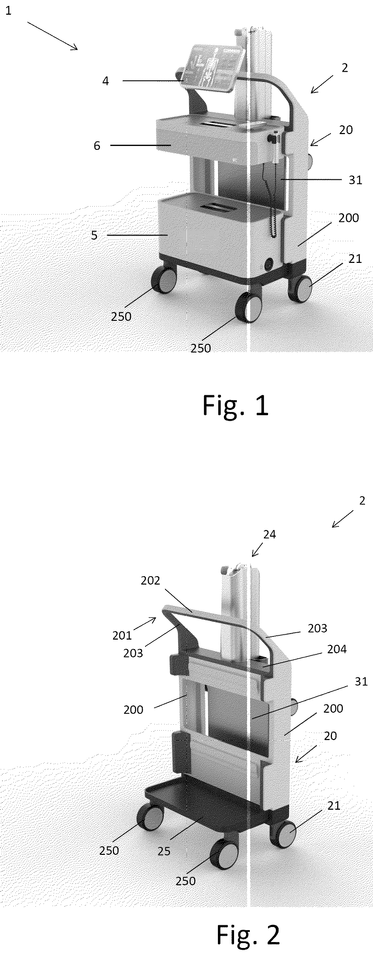

La figure 1 représente une partie d'un système d'imagerie médicale 1 selon un mode de réalisation de l'invention.

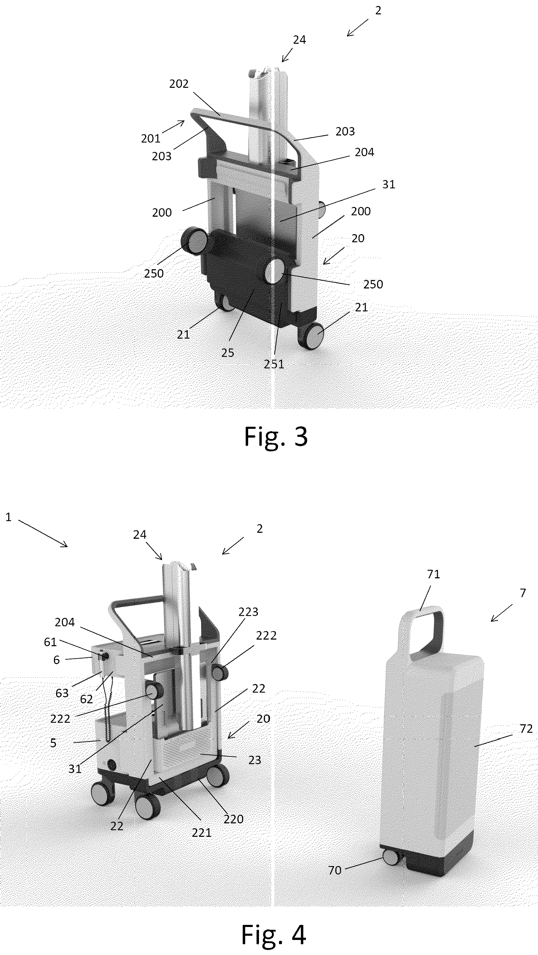

Le système d'imagerie médicale 1 comporte un support 2. Le support 2 est également représenté individuellement aux figures 2 et 3.

Le support 2 comporte un cadre 20 ou châssis. Le cadre ou châssis constitue un dispositif permettant l'assemblage d'éléments. Le cadre 20 comprend deux montants 200. Les montants 200 sont de préférence parallèles entre eux.

Le cadre 20 comprend ici en outre une portion de préhension 201. La portion de préhension 201 relie les montants 200. La portion de préhension 201 comprend une barre 202 prolongée de part et d'autre par deux portions de liaison 203. De préférence, la barre 202 est sensiblement orthogonale aux montants 200. Les portions de liaison 203 sont sensiblement orthogonales à la barre 202. Chaque portion de liaison 203 prolonge l'un des montants 202.

Le cadre 20 est de préférence métallique.

Le support 2 comporte au moins deux roues 21. Les roues 21 sont assemblées au cadre 20. Par exemple, une roue 21 est fixée à une extrémité de chaque montant 200. Les roues 21 permettent le déplacement du support 2.

Selon un exemple de réalisation, les roues 21 peuvent être assemblées de manière amovible au cadre 20. Par exemple, les roues 21 peuvent être fixées sur une base, elle-même assemblée de manière amovible au cadre 20.

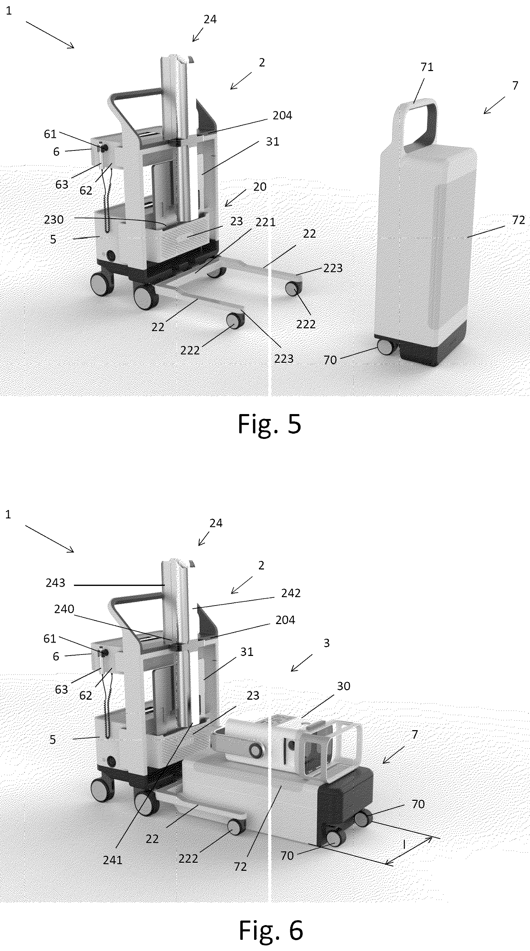

Le support 2 comporte en outre deux longerons 22 (mieux visibles en figures 4 et 5 par exemple). Les longerons 22 sont assemblés au cadre 20. Les longerons 22 sont montés en pivotement sur le cadre 20. Les longerons 22 sont configurés pour pivoter par rapport au cadre entre une position de fonctionnement et une position de rangement.

Le cadre 20 et les longerons 22 sont reliés par une charnière 220. Dans l'exemple représenté, le support 2 comporte une poutre de liaison 221 prolongeant orthogonalement les longerons 22. La poutre de liaison 221 est solidaire des deux longerons 22 et est reliée au cadre 20 par la charnière 220.

Les figures 1 à 4 montrent les longerons 22 en position de rangement. En position de rangement, les longerons 22 sont parallèles aux montants 200. Les longerons 22 s'étendent entre les montants 200. Chaque longeron 22 est en vis-à-vis avec l'un des montants 200. Cette configuration permet au support 2 de gagner en compacité.

Les figures 5 à 7 montrent les longerons 22 en position de fonctionnement. En position de fonctionnement, les longerons 22 s'étendent sensiblement horizontalement. Les longerons 22 s'étendent orthogonalement aux montants 200.

Chaque longeron comprend une roue 222 au niveau d'une extrémité libre 223. Les roues 222 facilitent le déplacement du support 2 lorsque les longerons 22 sont en position de fonctionnement. Le support 2 peut être tiré ou poussé, et ce quelle que soit la position dans laquelle se trouvent les longerons 22 (position de fonctionnement ou position de rangement).

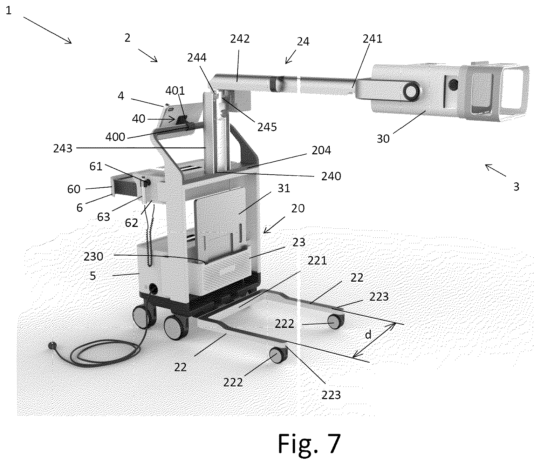

Le système d'imagerie médicale 1 comporte en outre un dispositif d'imagerie médicale 3 (visible en entier sur les figures 6, 7 et en partie sur les autres figures). De préférence, le dispositif d'imagerie médicale 3 est un dispositif de radiographie X. Le dispositif d'imagerie médicale 3 comporte ici une source 30 de rayons X. Cette source 30 fonctionne en association avec un détecteur 31 de rayons X. De préférence la source 30 de rayons X présente une limite supérieure de charge de tube supérieure ou égale à 40 mAs, ce qui permet alors de réaliser la totalité des examens souhaités.

Le dispositif d'imagerie médicale 3 peut être cependant différent par exemple un dispositif de tomographie ou d'échographie.

Le support 2 comporte, dans l'exemple de réalisation représenté, un bac de réception 23. Le bac de réception 23 comprend un logement 230 configuré pour recevoir le détecteur 31. Le bac de réception 23 présente ici une forme parallélépipédique. Le bac de réception 23 est fixé aux montants 200 du cadre 20. Alternativement, le bac de réception 23 peut être formé d'un seul tenant avec les montants du cadre 20.

Le bac de réception 23 s'étend de préférence entre les montants 200 du cadre 20. Cette configuration permet également au support 2 de gagner en compacité.

Le support 2 comporte également un bras 24. Le bras 24 est assemblé au cadre 20. Le bras 24 comprend une extrémité fixe 240 assemblée au cadre 20 et une extrémité libre 241. L'extrémité fixe 240 peut être fixée sur une barre de fixation 204 du cadre 20 s'étendant orthogonalement aux montants 200. L'extrémité libre 241 est configurée pour recevoir la source 30 de rayons X.

Le bras 24 comprend un tronçon avant 242 et un tronçon arrière 243. Le tronçon arrière 243 est fixé à la barre de fixation 204 par l'extrémité fixe 240. Le tronçon avant 242 comprend l'extrémité libre 241.

Le tronçon avant 242 et le tronçon arrière 243 sont articulés l'un par rapport à l'autre. Le tronçon avant 242 et le tronçon arrière 243 sont articulés autour d'un axe de pivotement 244 (bien visible en figure 7). Le bras 24 est mobile entre une position de service et une position escamotée. Le tronçon avant 242 et le tronçon arrière 243 sont repliés l'un sur l'autre lorsque le bras 24 est en position escamotée, comme visible sur les figures 1 à 6. Le tronçon avant 242 et le tronçon arrière 243 forment ensemble un angle non nul lorsque le bras 24 est en position de service, de préférence supérieur à un angle prédéfini (par exemple supérieur à un angle de 30°). Par exemple comme visible en figure 7, l'angle formé entre le tronçon avant 242 et le tronçon arrière 243 est sensiblement droit.

Le bras 24 peut être mobile entre la position de service et la position escamotée manuellement ou automatiquement. Par exemple, le système d'imagerie médicale 1 comporte en outre un actionneur 245 assemblé au bras 24. L'actionneur 245 est configuré pour déplacer le bras 24 entre la position de service et la position escamotée. L'actionneur 245 permet de positionner le bras 24 à la hauteur souhaitée pour réaliser l'examen médical. L'actionneur 245 est de préférence un vérin électrique.

Grâce au bras 24, la source 31 de rayons X est assemblée au support de manière amovible. L'assemblage est réalisé au niveau de l'extrémité libre 241 du bras 24. L'assemblage amovible peut être effectué par tout moyen de fixation amovible, typiquement clipsage, emboîtage élastique, boulonnage, etc.

L'extrémité libre 241 comprend avantageusement un système d'alignement secondaire permettant, avec un système d'alignement primaire de la source 31 de rayons, un alignement optimisé de la source 31 de rayons X avec la cible (à savoir un patient et le détecteur 30). Le système d'alignement primaire et le système d'alignement secondaire sont par exemple magnétiques.

L'utilisation d'un actionneur limite les efforts du manipulateur/personnel médical. En effet, ce dernier n'a pas à soulever le bras 24 avec la source 30 de rayons pour le positionner en position de service.

Le système d'imagerie médicale 1 comporte en outre un dispositif de commande 4. Le dispositif de commande 4 est configuré pour commander le dispositif d'imagerie médicale 3.

De préférence, le dispositif de commande 4 est également configuré pour lire et afficher une image reçue depuis le dispositif d'imagerie médicale 3.

Le dispositif de commande 4 comprend typiquement un outil informatique de gestion de données et d'images. Le dispositif de commande 4 peut comporter ou être une tablette, notamment une tablette tactile (comme sur l'exemple représenté) ou un ordinateur. Le dispositif de commande 4 comprend notamment un afficheur pour afficher l'image reçue.

Le dispositif de commande 4 est assemblé de manière amovible au support 2. Le dispositif de commande 4 peut être assemblé par tous moyens amovibles, tels que le clipsage, emboîtage élastique, boulonnage, etc. Le dispositif de commande 4 est ici assemblé à la portion de préhension 201 du cadre 20. En particulier, le dispositif de commande 4 est assemblé à la barre 202. Le dispositif de commande 4 est ici assemblé sur une pièce de fixation 40 (visible en figure 7) elle-même assemblée à la barre 202. La pièce de fixation 40 comprend une portion d'attache 400 sous forme de crochet conformée à la barre 202. La portion d'attache 400 est élastique. La pièce de fixation 40 comprend par ailleurs une portion de support 401 prolongeant la portion d'attache 400. La portion de support 401 est configurée pour permettre l'assemblage du dispositif de commande 4, ici la tablette. Le dispositif de commande 4 peut être par exemple clipsé ou assemblé magnétiquement à la portion de support 401.

Le dispositif de commande 4 peut être aussi réuni à (ou disposé sur) un boîtier 6 décrit plus loin, sans utilisation de pièce de fixation.

Le système d'imagerie médicale 1 comporte également un dispositif d'alimentation électrique 5. Le dispositif d'alimentation électrique comprend de préférence au moins une batterie. Le dispositif d'alimentation électrique 5 est configuré pour alimenter en énergie électrique des composants électriques du système d'imagerie médicale 1, par exemple le dispositif d'imagerie médicale 3 et/ou le dispositif de commande 4. Le dispositif d'alimentation électrique 5 peut alimenter tout ou partie des composants électriques du système d'imagerie médicale 1.

Le dispositif d'alimentation électrique 5 est assemblé de manière amovible au support 2.

Dans l'exemple représenté, le support 2 comporte une plaque de réception 25 (bien visible à la figure 2). La plaque de réception 25 est configurée pour recevoir le dispositif d'alimentation électrique 5. La plaque de réception 25 présente pour cela des dimensions et une forme similaire à celles du dispositif d'alimentation électrique 5.

La plaque de réception 25 est assemblée au cadre 20, par exemple par une liaison charnière. La plaque de réception 25 est configurée pour pivoter entre une position de fonctionnement et une position de rangement.

Les figures 1, 2 et 4 à 7 montrent la plaque de réception 25 en position de fonctionnement. La plaque de réception 25 s'étend sensiblement horizontalement. La plaque de réception 25 est orthogonale aux montants 200 du cadre 20. La plaque de réception 25 en position de fonctionnement est apte à recevoir la batterie 5.

La figure 3 montre la plaque de réception 25 en position de rangement. La plaque de réception 25 s'étend sensiblement verticalement. La plaque de réception 25 est orthogonale aux montants 200 du cadre 20.

La plaque de réception 25 permet à la batterie 5 d'être assemblée ou montée de manière amovible au support 2.

La plaque de réception 25 comprend de préférence au moins une roue 250. La plaque de réception 25 comprend ici deux roues 250. Les roues 250 sont fixées sur une face inférieure 251 de la plaque de réception 25. Les roues 250 facilitent le déplacement du support 2 lorsque la plaque de réception 25 est en position de fonctionnement. Le support 2 peut être tiré ou poussé, et ce quelle que soit la position de la plaque de réception 25 (position de fonctionnement ou position de rangement).

Dans l'exemple de réalisation où les roues 21 sont assemblées de manière amovible au cadre 20 (par exemple au moyen d'une base, elle-même assemblée de manière amovible au cadre 20), le dispositif d'alimentation électrique 5 est assemblé au cadre 20 par un système d'assemblage amovible (e.g. crochets, clips, ou tout autre moyen d'assemblage mécanique ou magnétique). Le système d'assemblage amovible est lui-même fixé au cadre 20, par exemple à la barre de fixation 204. Cette configuration a pour avantage de présenter une masse globale plus faible et un système d'imagerie médicale avec moins de roues. Par exemple, le système d'imagerie médicale peut avoir seulement quatre roues au total au lieu de six roues comme sur l'exemple de réalisation représenté sur les figures.

Le système d'imagerie médicale 1 peut comporter également un boîtier 6. Le boîtier 6 est configuré pour être assemblé au support 2 de manière amovible. Le boîtier 6 est ici clipsé à la barre de fixation 204. Le boîtier 6 peut être assemblé au support 2 autrement, par exemple par emboîtage élastique ou boulonnage.

Le boîtier 6 peut servir de rangement pour le personnel médical utilisant le système d'imagerie médicale 1. Le boîtier 6 comprend par exemple un tiroir 60 coulissant sur rails comme visible sur la figure 7. Le boîtier 6 comprend ici un support d'assemblage 61. Le support d'assemblage 61 est fixé sur une paroi latérale 62 du boîtier 6. Le support d'assemblage 61 est configuré pour recevoir une télécommande 63 de prise de vue. Le support d'assemblage 61 permet ainsi de ranger la télécommande 63 tout en la gardant facilement accessible en cas de besoin.

Avantageusement, le boîtier 6 présente une forme et/ou des dimensions identiques à celles de la batterie 5. Cela permet, si besoin de ranger ou transporter le boîtier 6 et la batterie 5, de les empiler et ainsi de faciliter le rangement/transport.

Le boîtier 6 peut aussi servir de support au dispositif de commande 4.

Le système d'imagerie médicale 1 comporte avantageusement également une valise 7 représentée sur les figures 4 à 6. La valise 7 est dimensionnée pour loger au moins la source 30 de rayons X et/ou le détecteur 31. La valise peut comprendre au moins un logement pour loger la source 30 de rayons X et/ou le détecteur 31. La valise 7 peut également loger le dispositif de commande 4. La valise 7 peut alors comprendre à cet effet un logement spécifique pour le dispositif de commande 4. La valise 7 peut aussi contenir d'autres éléments pouvant s'avérer utiles pour pratiquer l'examen d'imagerie médicale.

De préférence, la valise 7 présente une largeur l sensiblement égale à une distance d entre les longerons 22 du support 2. Dans l'exemple représenté les longerons 22 sont parallèles entre eux. Cela permet, comme visible sur la figure 6, de disposer la valise 7 entre les longerons 22 lorsque les longerons 22 sont en position de fonctionnement. La valise 7 ainsi dimensionnée est calée entre les longerons 22.

La valise 7 est de préférence munie de deux roues 70. La valise 7 comprend également de préférence une poignée 71. Le personnel médical ou manipulateur peut ainsi tirer et manipuler et aisément la valise 7.

Selon un autre exemple de réalisation alternatif ou complémentaire, la valise 7 peut comprendre des sangles permettant son transport à dos. La valise 7 peut ainsi être transportée comme un sac à dos.

Lors du transport ou du rangement du système d'imagerie médicale 1, les différentes parties (support, batterie, boîtier, etc.) de ce dernier peuvent être rangées de manière compacte. Le dispositif d'imagerie médicale 3, le dispositif de commande 4, le dispositif d'alimentation électrique 5 est séparé du support 2. Le boîtier 6 est également séparé du support 2 si le système d'imagerie médicale 1 comprend un boîtier. Chacune des parties du système d'imagerie médicale 1 peut ainsi être manipulée individuellement pour son rangement ou transport. Le système d'imagerie médicale 1 est dimensionné pour permettre son transport et rangement dans un coffre de voiture standard. Lors du transport ou rangement, les longerons 22 et la plaque de réception 25 sont dans la position de rangement, et le bras 24 est dans la position escamotée. Le système d'imagerie médicale 1 est ainsi dans une configuration dite de transport.

Afin de se déplacer au sein d'un même lieu, par exemple de chambre en chambre dans un hôpital, le système d'imagerie médicale 1 peut prendre une configuration de déplacement visible à la figure 4 selon un exemple particulier. Dans cette configuration, le dispositif de commande 4, la batterie 5 et le boîtier 6 sont assemblées au support 2. Le détecteur 31 de rayons X est logé dans le bac de réception 23. La source 30 de rayons X est rangée est dans la valise 7. Les longerons 22 sont dans la position de rangement. La plaque de réception 25 est dans la position de fonctionnement pour pouvoir recevoir la batterie 5. La configuration de déplacement permet au manipulateur/personnel médical de se déplacer facilement en poussant le support 2 et en tirant la valise 7. Un seul manipulateur peut déplacer facilement et avec peu d'effort le système d'imagerie médicale 1.

Lorsque le manipulateur arrive au lieu d'examen médical, notamment près du patient, il peut déployer facilement le système d'imagerie médicale 1. Les longerons 22 sont d'abord passés en position de fonctionnement, comme sur la figure 5. La valise 7 est ensuite disposée entre les longerons 22 comme sur la figure 6. Un couvercle 72 de la valise 7 est ouvert et la source 30 de rayons X est sortie. Le couvercle 72 est éventuellement refermé puis la source 30 de rayons X est posée sur le couvercle 72 refermé. La figure 6 montre le couvercle 72 fermée et la source 30 de rayons X sortie de la valise 7 et disposée sur le couvercle 72. Le bras 24 jusqu'alors en position escamotée est déployé, par exemple au moyen de l'actionneur 245, pour se déplacer jusqu'à la source 30 de rayons X. Le bras 24 peut être assemblé automatiquement à la source 30 de rayons X. Le bras 24 prend enfin la position de service comme, par exemple celle montrée sur la figure 7. Le système d'imagerie médicale 1 est ainsi dans une configuration dite déployée. Il est à noter que la valise 7 n'a pas été représentée sur la figure 7 mais celle-ci peut être gardée entre les longerons 22 si besoin en configuration déployée (par exemple en cas de besoin en cours d'examen médical d'objets logés dans la valise).

Après déploiement du système d'imagerie médicale 1, le détecteur 31 de rayons X est sorti du bac de réception 23 afin d'être positionné derrière le patient pour une prise de vue.

Le manipulateur peut disposer le système d'imagerie médicale 1 dans les différentes configurations (configuration de transport, configuration de déplacement et configuration déployée) aisément et sans effort. Toutes les manipulations (rangement, transport, déploiement, etc.) peuvent être faites par une seule personne.

Le système d'imagerie médicale selon l'invention est modulaire, ergonome, compacte, léger tout en permettant un large panel d'examens médicaux.

## Revendications

1. Système d'imagerie médicale comportant :
- un support (2) comportant un cadre (20) ;
- au moins deux roues (21) assemblées directement ou indirectement au cadre (20) de sorte à permettre le déplacement dudit support (2) ;
- un dispositif d'imagerie médicale (3) ;
- un dispositif de commande (4) configuré pour commander le dispositif d'imagerie médicale (3) ; et
- un dispositif d'alimentation électrique (5) configuré pour alimenter en énergie électrique le dispositif d'imagerie médicale (3) et/ou le dispositif de commande (4),
le dispositif d'imagerie médicale (3), le dispositif de commande (4) et le dispositif d'alimentation électrique (5) étant configurés pour être chacun assemblé au support (2) de manière amovible,
le système d'imagerie médicale étant **caractérisé en ce que** le support (2) comporte en outre deux longerons (22) assemblés au cadre (20) et configurés pour pivoter par rapport au cadre entre une position de fonctionnement et une position de rangement, le système d'imagerie médicale comportant en outre une source (30) de rayons X, un détecteur (31) de rayons X et une valise (7), la valise (7) étant dimensionnée pour loger la source (30) de rayons X et/ou le détecteur (31) de rayons X, la valise (7) présentant une largeur sensiblement égale à la distance entre les longerons (22).

2. Système d'imagerie médicale selon la revendication 1, comportant en outre un boîtier (6) configuré pour être assemblé au support (2) de manière amovible.

3. Système d'imagerie médicale selon la revendication 1 ou la revendication 2, dans lequel les roues (21) sont assemblées au cadre (20) de manière amovible.

4. Système d'imagerie médicale selon l'une des revendications 1 à 3, dans lequel les roues (21) sont assemblées à une base, ladite base étant assemblée de manière amovible au cadre (20).

5. Système d'imagerie médicale selon l'une des revendications 1 à 4, dans lequel le cadre (20) comporte deux montants (200) et une portion de préhension (201) reliant les montants (200).

6. Système d'imagerie médicale selon la revendication 5, dans lequel les longerons (22) en position de fonctionnement s'étendent sensiblement horizontalement, les longerons (22) en position de rangement étant parallèles aux montants (200) du cadre (20) et s'étendant entre lesdits montants (200).

7. Système d'imagerie médicale selon la revendication 6, dans lequel chaque longeron (22) comprend une roue (222) au niveau d'une extrémité libre (223).

8. Système d'imagerie médicale selon l'une des revendications 5 à 7, dans lequel le support (2) comporte en outre une plaque de réception (25) configurée pour recevoir le dispositif d'alimentation électrique (5), la plaque de réception (25) étant assemblée au cadre (20) et configurée pour pivoter par rapport au cadre entre une position de fonctionnement et une position de rangement, la plaque de réception (25) en position de fonctionnement s'étendant sensiblement horizontalement, la plaque de réception (25) en position de rangement étant orthogonale aux montants du cadre (20) et s'étendant sensiblement verticalement.

9. Système d'imagerie médicale selon la revendication 8, dans lequel la plaque de réception (25) comprend au moins une roue (250) sur une face inférieure (251).

10. Système d'imagerie médicale selon l'une des revendications 1 à 9, dans lequel la source (30) de rayons X présente une limite supérieure de charge de tube supérieure ou égale à 40 mAs.

11. Système d'imagerie médicale selon l'une des revendications 1 à 10, dans lequel le support (2) comprend un logement (230) configuré pour recevoir le détecteur (31) de rayons X.

12. Système d'imagerie médicale selon l'une des revendications 1 à 11, dans lequel le support (2) comporte un bras (24) assemblé au cadre (20) et ayant une extrémité libre (241) configurée pour recevoir la source (30) de rayons X.

13. Système d'imagerie médicale selon la revendication 12, dans lequel le bras (24) comprend un tronçon avant (242) et un tronçon arrière (243) articulés l'un par rapport à l'autre, le bras (24) étant mobile entre une position de service et une position escamotée, le tronçon avant (242) et le tronçon arrière (243) formant ensemble un angle non nul, de préférence supérieur à 30°, lorsque le bras (24) est en position de service, le tronçon avant (242) et le tronçon arrière (243) étant repliés l'un sur l'autre lorsque le bras (24) est en position escamotée.

14. Système d'imagerie médicale selon la revendication 13, comportant en outre un actionneur (245) assemblé au bras (24) et configuré pour déplacer le bras (24) entre la position de service et la position escamotée, l'actionneur (245) étant de préférence un vérin électrique.

## Patentansprüche

1. Medizinisches Bildgebungssystem, das umfasst:
- einen Träger (2), der einen Rahmen (20) umfasst;
- mindestens zwei Räder (21), die direkt oder indirekt am Rahmen (20) montiert sind, um eine Bewegung des Trägers (2) zu ermöglichen;
- eine medizinische Bildgebungsvorrichtung (3);
- eine Steuereinheit (4), die konfiguriert ist, um die medizinische Bildgebungsvorrichtung (3) zu steuern; und
- eine Stromversorgungsvorrichtung (5), die konfiguriert ist, um die medizinische Bildgebungsvorrichtung (3) und/oder die Steuereinheit (4) mit elektrischem Strom zu versorgen,
wobei die medizinische Bildgebungsvorrichtung (3), die Steuereinheit (4) und die Stromversorgungsvorrichtung (5) konfiguriert sind, um jeweils abnehmbar am Träger (2) montiert zu werden,
wobei das medizinische Bildgebungssystem **dadurch gekennzeichnet ist, dass** der Träger (2) weiter zwei Holme (22) umfasst, die am Rahmen (20) montiert sind und konfiguriert sind, um in Bezug zum Rahmen zwischen einer Betriebsposition und einer Aufbewahrungsposition zu schwenken, wobei das medizinische Bildgebungssystem weiter eine Röntgenstrahlenquelle (30), einen Röntgenstrahlendetektor (31) und einen Koffer (7) umfasst, wobei der Koffer (7) bemessen ist, um die Röntgenstrahlenquelle (30) und/oder den Röntgenstrahlendetektor (31) aufzunehmen, wobei der Koffer (7) eine im Wesentlichen gleiche Breite wie der Abstand zwischen den Holmen (22) aufweist.

2. Medizinisches Bildgebungssystem nach Anspruch 1, das weiter ein Gehäuse (6) umfasst, das konfiguriert ist, um abnehmbar am Träger (2) montiert zu werden.

3. Medizinisches Bildgebungssystem nach Anspruch 1 oder Anspruch 2, wobei die Räder (21) abnehmbar am Rahmen (20) montiert sind.

4. Medizinisches Bildgebungssystem nach einem der Ansprüche 1 bis 3, wobei die Räder (21) an einer Basis montiert sind, wobei die Basis abnehmbar am Rahmen (20) montiert ist.

5. Medizinisches Bildgebungssystem nach einem der Ansprüche 1 bis 4, wobei der Rahmen (20) zwei Ständer (200) und einen Greifabschnitt (201) umfasst, der die Ständer (200) verbindet.

6. Medizinisches Bildgebungssystem nach Anspruch 5, wobei sich die Holme (22) in Betriebsposition im Wesentlichen horizontal erstrecken, die Holme (22) in Aufbewahrungsposition parallel zu den Ständern (200) des Rahmens (20) verlaufen und sich zwischen den Ständern (200) erstrecken.

7. Medizinisches Bildgebungssystem nach Anspruch 6, wobei jeder Holm (22) ein Rad (222) im Bereich eines freien Endes (223) umfasst.

8. Medizinisches Bildgebungssystem nach einem der Ansprüche 5 bis 7, wobei der Träger (2) weiter eine Aufnahmeplatte (25) umfasst, die konfiguriert ist, um die Stromversorgungsvorrichtung (5) aufzunehmen, wobei die Aufnahmeplatte (25) am Rahmen (20) montiert, und konfiguriert ist, um in Bezug zum Rahmen zwischen einer Betriebsposition und einer Aufbewahrungsposition zu schwenken, wobei sich die Aufnahmeplatte (25) in Betriebsposition im Wesentlichen horizontal erstreckt, die Aufnahmeplatte (25) in Aufbewahrungsposition orthogonal zu den Ständern des Rahmens (20) verlaufen, und sich im Wesentlichen vertikal erstrecken.

9. Medizinisches Bildgebungssystem nach Anspruch 8, wobei die Aufnahmeplatte (25) mindestens ein Rad (250) auf einer Unterseite (251) umfasst.

10. Medizinisches Bildgebungssystem nach einem der Ansprüche 1 bis 9, wobei die Röntgenstrahlenquelle (30) eine obere Röhrenbelastungsgrenze größer oder gleich 40 mAs aufweist.

11. Medizinisches Bildgebungssystem nach einem der Ansprüche 1 bis 10, wobei der Träger (2) eine Aufnahme (230) umfasst, die konfiguriert ist, um den Röntgenstrahlendetektor (31) aufzunehmen.

12. Medizinisches Bildgebungssystem nach einem der Ansprüche 1 bis 11, wobei der Träger (2) einen Arm (24) umfasst, der am Rahmen (20) montiert ist und ein freies Ende (241) aufweist, das konfiguriert ist, um die Röntgenstrahlenquelle (30) aufzunehmen.

13. Medizinisches Bildgebungssystem nach Anspruch 12, wobei der Arm (24) ein vorderes Teilstück (242) und ein hinteres Teilstück (243) umfasst, die relativ zueinander gelenkig gelagert sind, wobei der Arm (24) zwischen einer Serviceposition und einer eingefahrenen Position bewegbar ist, wobei das vordere Teilstück (242) und das hintere Teilstück (243) zusammen einen Winkel ungleich null, vorzugsweise größer als 30°, bilden, wenn der Arm (24) in Serviceposition ist, wobei das vordere Teilstück (242) und das hintere Teilstück (243) zusammengeklappt sind, wenn der Arm (24) in eingefahrener Position ist.

14. Medizinisches Bildgebungssystem nach Anspruch 13, das weiter eine Betätigungsvorrichtung (245) umfasst, das am Arm (24) montiert ist, und konfiguriert ist, um den Arm (24) zwischen der Serviceposition und der eingefahrenen Position zu bewegen, wobei die Betätigungsvorrichtung (245) vorzugsweise ein Elektrozylinder ist.

## Claims

1. Medical imaging system comprising:
- a support (2) comprising a frame (20);
- at least two wheels (21) assembled directly or indirectly to the frame (20) so as to allow said support (2) to be moved;
- a medical imaging device (3);
- a control device (4) configured to control the medical imaging device (3); and
- an electric power supply device (5) configured to supply electrical energy to the medical imaging device (3) and/or the control device (4),
the medical imaging device (3), the control device (4) and the electric power supply device (5) each being configured to be removably assembled to the support (2),
the medical imaging system being **characterized in that** the support (2) further comprises two outriggers (22) assembled to the frame (20) and configured to pivot with respect to the frame between an operational position and a storage position, the medical imaging system further comprising an X-ray source (30), an X-ray detector (31) and a case (7), the case (7) being dimensioned to house the X-ray source (30) and/or the X-ray detector (31), the case (7) having a width substantially equal to the distance between the outriggers (22).

2. Medical imaging system according to claim 1, further comprising a housing (6) configured to be removably assembled to the support (2).

3. Medical imaging system according to claim 1 or claim 2, wherein the wheels (21) are removably assembled to the frame (20).

4. Medical imaging system according to one of claims 1 to 3, wherein the wheels (21) are assembled to a base, said base being removably assembled to the frame (20).

5. Medical imaging system according to one of claims 1 to 4, wherein the frame (20) comprises two riser sections (200) and a grip portion (201) connecting the riser sections (200).

6. Medical imaging system according to claim 5, wherein the outriggers (22) in the operating position extend substantially horizontally, the outriggers (22) in the storage position being parallel to the riser sections (200) of the frame (20) and extending between said riser sections (200).

7. Medical imaging system according to claim 6, wherein each outrigger (22) comprises a wheel (222) at a free end (223).

8. Medical imaging system according to one of claims 5 to 7, wherein the support (2) further comprises a receiving plate (25) configured to receive the electric power supply device (5), the receiving plate (25) being assembled to the frame (20) and configured to pivot with respect to the frame between an operating position and a storage position, the receiving plate (25) in the operating position extending substantially horizontally, the receiving plate (25) in the storage position being orthogonal to the riser sections of the frame (20) and extending substantially vertically.

9. Medical imaging system according to claim 8, wherein the receiving plate (25) comprises at least one wheel (250) on a lower face (251).

10. Medical imaging system according to one of claims 1 to 9, wherein the X-ray source (30) has an upper tube charge limit greater than or equal to 40 mAs.

11. Medical imaging system according to one of claims 1 to 10, wherein the support (2) comprises a housing (230) configured to receive the X-ray detector (31).

12. Medical imaging system according to one of claims 1 to 11, wherein the support (2) comprises an arm (24) assembled to the frame (20) and having a free end (241) configured to receive the X-ray source (30).

13. Medical imaging system according to claim 12, wherein the arm (24) comprises a front section (242) and a rear section (243) articulated relative to one another, the arm (24) being mobile between a service position and a retracted position, the front section (242) and the rear section (243) together forming a non-zero angle, preferably greater than 30°, when the arm (24) is in the service position, the front section (242) and the rear section (243) being folded one over the other when the arm (24) is in the retracted position.

14. Medical imaging system according to claim 13, further comprising an actuator (245) assembled to the arm (24) and configured to move the arm (24) between the service position and the retracted position, the actuator (245) being preferably an electric cylinder.
